# EUROPÄISCHE PATENTANMELDUNG

(11) **EP 2 138 160 A2**
(43) Veröffentlichungstag der Anmeldung: **30.12.2009**
(21) Anmeldenummer: 09160962.8
(22) Anmeldetag: 25.05.2009
(51) Int. Cl.: A61K 8/891, A61K 8/73, A61K 8/63, A61K 8/39, A61K 8/42, A61K 8/44, A61Q 5/12

(54) **Haarspülung mit Silikonemulsion**

(30) Priorität: 27.06.2008 DE 102008030139
(71) Anmelder: Beiersdorf AG, 20245 Hamburg (DE)
(72) Erfinder: Demitz, Michael, 22529, Hamburg (DE); Köhler, Manuela, 20144, Hamburg (DE); Saladin, Sandra, 20144, Hamburg (DE); Mahadeshwar, Anand, 22529, Hamburg (DE); Wendicke, Silke, 22303, Hamburg (DE)

(57) **Zusammenfassung**

Kosmetische Haarbehandlungszubereitung vorgesehen für das Ausspülen nach der Anwendung mit kationischen Polymeren in Gehalten von 0,4 bis 0,7 Gew.-% vorhanden in einer hochmolekularen Silikonemulsion mit Partikelgrößen von 10 bis 50 Mikrometern.

## Beschreibung

Haarspülungen werden zur Pflege nach der Reinigung der Haare angewendet. Ein wichtiger Faktor ist die Sensorik der Spülung während des Auftragens auf das Haar. Der Konsument verbindet mit einer reichhaltigen Haarspülung eine sehr gute Pflegewirkung. Viele Haarspülungen zeigen zwar eine gute Leistung hinsichtlich der Pflegeeigenschaften, haben jedoch eine schlechte Sensorik, vor allem im nassen Haar.

"Sensorik" im Sinne dieser Schrift umfasst alle fühlbaren und für den Anwender relevanten Eigenschaften des Produktes bei Entnahme des Produktes aus der Packung, bestimmungsgemäßer Anwendung (Einmassieren in das Haar, Aufenthalt des Produktes im Haar, Ausspülen) sowie die während und nach der Anwendung fühlbaren Veränderungen an Haar, Kopfhaut und Fingern. Insbesondere umfasst "Sensorik" die Eigenschaften "Gehaltvoll", "Gleitvermögen", "Entwirrbarkeit", "Kämmbarkeit", "Haarstruktur", "Weich geschmeidig".

"Gehaltvoll" im Sinne dieser Schrift wird wie folgt festgelegt: Es wird gemessen in welchem Ausmaß das Produkt als Schicht/Film zwischen der Haarsträhne und den Fingern wahrnehmbar ist. Hierbei wird eine 10er Skala verwendet bei der 10 den höchstmöglichen Gehalt darstellt.

"Gleitvermögen" im Sinne dieser Schrift bedeutet das Gleiten der Finger mit mittlerem/gleich bleibendem Druck an der Haarsträhne von oben nach unten. Hierbei wird eine 10er Skala verwendet bei der 10 das höchstmögliche Gleitvermögen darstellt.

"Entwirrbarkeit" im Sinne dieser Schrift ist die Messung wie leicht sich die Haare nach dem Ausspülen des Produkts mit dem Kamm entwirren lassen. Die Haare sind dann entwirrt, wenn der Kamm einmal komplett von oben nach unten durch die Haarsträhne geführt werden kann. Hierbei wird eine 10er Skala verwendet bei der 10 die höchstmögliche Entwirrbarkeit darstellt.

"Kämmbarkeit" im Sinne dieser Schrift ist die Messung wie leicht sich das Haar mit dem Kamm durchkämmen lässt, nachdem die Haare entwirrt wurden. Hierbei wird eine 10er Skala verwendet bei der 10 die höchstmögliche Kämmbarkeit darstellt.

"Haarstruktur" im Sinne dieser Schrift bedeutet die Beurteilung des Haares durch horizontales Rollen der Haare zwischen Daumen und Zeigefinger/ Mittelfinger einer Hand. Dies wird an verschiedenen Stellen der Strähne beginnend von oben nach unten geprüft. Hierbei wird eine 10er Skala verwendet bei der 10 die höchstmögliche Haarstruktur darstellt.

"Weich geschmeidig" im Sinne dieser Schrift bedeutet das sensorische Empfinden nach Applikation des Produktes im nassen und trockenen Haar durch einen Experten Panel.

Silikonemulsionen sind Zubereitungen, die in Wasser dispergierte oder emulgierte Silikonöle enthalten. Solche Silikonöle sind beispielsweise Polidimethylsiloxane. Diese sind oft durch oberflächenaktive Substanzen dispergiert oder emulgiert. Geeignete Emulgatoren sind Cocoamidopropylbettaoin, C12-C15 Pareth-3 oder Guarhydroxytrimoniumchlorid. Die Tröpchengrößen bewegen sich im Bereich einiger Mikrometer, insbesondere im Bereich von 15-30 Mikrometern.

Kationische Polymere sind Makromolekulare Substanzen, welche in der Haupt- und/oder Seitenkette eine Gruppe aufweisen, welche "temporär" oder "permanent" kationisch sein kann.

Geeignete kationische Polymere sind beispielsweise quaternisierte Cellulose-Derivate, wie sie unter den Bezeichnungen Celquat und Polymer JR im Handel erhältlich sind. Die Verbindungen Celquat H 100, Celquat L 200 und Polymer JR 400 sind bevorzugte quaternierte Cellulose-Derivate, kationische Alkylpolyglycoside gemäss der DE 44 13 686, kationisierter Honig, beispielsweise das Handelsprodukt Honeyquat 50, kationische Guar-Derivate, wie insbesondere die unter den Handelsnamen Cosmedia Guar und Jaguarvertriebenen Produkte, polymere Dimethyldiallylammoniumsalze und deren Copolymere mit Estern und Amiden von Acrylsäure und Methacrylsäure. Die unter den Bezeichnungen Merquat 100 (Poly(dimethyldiallylammoniumchlorid)) und Merquat550 (Dimethyldiallylammoniumchlorid-Acrylamid-Copolymer) im Handel erhältlichen Produkte sind Beispiele für solche kationischen Polymere, Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat 734 und Gafquat 755 im Handel erhältlich, Vinylpyrrolidon-Vinylimidazoliummethochlorid-Copolymere, wie sie unter den Bezeichnungen Luviquat FC 370, FC 550, FC 905 und HM 552 angeboten werden, quaternierter Polyvinylalkohol, sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette, die unter den Bezeichnungen Polyquaternium-24 (Handelsprodukt z. B. Quatrisoft LM 200), bekannten Polymere, die Copolymere des Vinylpyrrolidons, wie sie als Handelsprodukte Copolymer 845 (Hersteller: ISP), Gaffix VC 713 (Hersteller: ISP), Gafquat ASCP 1011, GafquatHS 110, Luviquat8155 und Luviquat MS 370 erhältlich sind.

EP1552807 offenbart Haarkonditionierzubereitungen mit kationisiertem Guar und bestimmten Dialkylammoniumsalzen.

DE102005028763 offenbart Haarbehandlungsmittel mit quaternierten Fettsäureamidoaminen und kationischen Polymeren.

WO99/34768 offenbart Haarkonditionierzubereitungen mit bestimmten neutralisierten Fettsäureamidoaminen und bestimmten Silikonen.

Eine Haarspülung soll aus Konsumentensicht reichhaltig und cremig sein, das Haar pflegen, das Haar im nassen und trockenen Zustand weich, geschmeidig, gut kämmbar, glänzend machen und die Spülung muss schnell wirken.

Wie in der Stand der Technik beschrieben können kationische Polymere wie beispiels-weise kationisches Guar in Haarpflegeformulierungen die kationische Tenside und Fett-alkohol enthalten, zugefügt werden, um die konditionierenden Eigenschaften zu ver-bessern, allerdings gibt es einen Nachteil hinsichtlich der Sensorik im nassen Zustand; die Spülung fühlt sich auf dem Haar wässriger an. Durch die Verwendung einer Silikon-emulsion, die hochmolekulares Dimethicon und kationisches Polymer wie Guar Hydroxypropyltrimonium Chloride enthält, ist eine verbesserte Konditionierleistung des kationischen Pflegepolymers ohne die reichhaltige Sensorik des Conditioners zu verschlechtern, zu erwarten. Dies zeigte sich anhand eines Expertenpaneltests. Desweiteren führen geringe Mengen an Silikon zu einer überragenden Pflegeleistung wenn das kationische Polymer in der Emulsion vorliegt (im Vergleich zur Emulsion ohne Polymer).

Daher hat sich überraschend gezeigt, dass eine kosmetische Haarbehandlungszubereitung vorgesehen für das Ausspülen nach der Anwendung mit kationischen Polymeren in Gehalten von 0,4 bis 0,7 Gew.-% vorhanden in einer hochmolekularen Silikonemulsion mit Partikelgrößen von 10 bis 50 Mikrometern den Nachteilen des Standes der Technik abhilft.

Dabei ist es von Vorteil, wenn zusätzlich 0,1 bis 1 Gew.-% Oryzanol enthalten sind. Weiter ist es von Vorteil, wenn als kationisches Polymer Guar Hydroxypropyltrimonium Chlorid verwendet wird. Weiter ist es von Vorteil, wenn das kationisches Polymer Guar Hydroxypropyltrimonium Chloride eine mittlere Ladungsdichte von 0.2 bis 1 meq per gram hat. Solche Polymere sind Jaguar C-17(Rhodia Inc.) Jaguar C-1000(Rhodia Inc.) Jaguar C-13S(Rhodia Inc.) Jaguar C-14S(Rhodia Inc.) Jaguar Excel(Rhodia Inc.). Weiter ist es von Vorteil, wenn die Silikonemulsion eine Viskosität 6000 -12000 mPas (Brookfield, Spindel 6, 50 RPM) hat. Eine typische Silikon-emulsion weist einen pH von 5.0 -7.0, eine Partikelgröße von: 15.0 - 30.0 µm, einen Gehalt an Phenoxyethanol von 0.85 % - 0.95 %, eine Dichte von 0.9750 - 0.9900 kg/I, eine Silikonviskosität von 500.00cst auf und enthält als Emulgator C12-15 Pareth-3. Weiter ist es von Vorteil, wenn die Zubereitung frei von Cellulosederivaten ist. Weiter ist es von Vorteil, wenn die Zubereitung nicht waschaktiv ist. Weiter ist es von Vorteil, wenn die Zubereitung frei von anionischen Tensiden ist. Weiter ist es von Vorteil, die Zubereitung außer kationischen keine weiteren Polymere enthält. Weiter ist es von Vorteil, wenn die Zubereitung amphotere Tenside enthält, bevorzugt Betaine, ganz besonders bevorzugt Cocobetaine. Die Konzentration dieser amphoteren Tenside beträgt vorteilhaft 0.25 Gew.-% bis 1 Gew.-% Aktivgehalt.

Erfindungsgemäße Zubereitungen können zusätzlich UV- Filter, Polyole, Konservierungsmittel, hydrolysierte Proteine, Pflanzenextrakte, Parfümöle, und Antioxidantien sowie auch amphotere Tenside enthalten.

Besonders bevorzugte erfindungsgemäße Mittel Fasern sind dadurch gekennzeichnet, dass sie als Pflegestoff - bezogen auf ihr Gewicht - 0,0001 bis 1 Gew.-%, bevorzugt 0,001 bis 0,5 Gew.-% und besonders bevorzugt 0,005 bis 0,1 Gew.-% mindestens eines Ubichinons und/oder mindestens eines Ubichinols und/oder mindestens eines Derivates dieser Substanzen enthalten, wobei bevorzugte Mittel Coenzym Q10 enthalten.

Alternativ zu den besonders bevorzugten Ubichinonen oder zusätzlich zu ihnen können die erfindungsgemäßen Mittel auch Plastochinone enthalten. Hier sind bevorzugte erfindungsgemäße Mittel dadurch gekennzeichnet, dass sie 0,0002 bis 4 Gew.-%, vorzugsweise 0,0005 bis 3 Gew.-%, besonders bevorzugt 0,001 bis 2 Gew.-%, weiter bevorzugt 0,0015 bis 1 und insbesondere 0,002 bis 0,5 Gew.-% mindestens eines Plastochinons, besonders bevorzugt Mittel Plastochinon PQ-9 enthalten.

Es ist bekannt dass besonders tertiäre Amine in Kombination mit den Fettalkoholen Cetyl und Stearyl Alkoholen zu reichhaltigen, cremigen Spülungsgrundlagen führen, wie in EP 0859587B1, EP617953B1, WO2002102334A2 und EP1586298B1 beschrieben. Amidoaminenthaltige Spülungsformulierungen sind in Tabelle 1 abgebildet.

### Beispiele und Bestimmung der sensorischen Eigenschaften

**Tabelle 1**

| | wt% | **1** | **2** | **3** |
|---|---|---|---|---|
| 1 | Methylparaben | 0,25 | 0,25 | 0,25 |
| 2 | Propylparaben | 0,10 | 0,10 | 0,10 |
| 3 | Aqua | Ad. 100 | Ad. 100 | Ad. 100 |
| 5 | Cetyl Alcohol | 2,50 | 2,50 | 2,50 |
| 6 | Lactic Acid | 0,78 | 0,78 | 0,78 |
| 7 | Sodium Chloride | 0,01 | 0,01 | 0,01 |
| 8 | Stearyl Alcohol | 4,80 | 4,80 | 4,80 |
| 9 | Oryzanol | 0,05 | 0,05 | 0,05 |
| 10 | Guar Hydroxypropyltrimonium Chloride | | 0,025 | 0,100 |
| 11 | Coco Betaine | 0,85 | 0,85 | 0,85 |
| 12 | Stearamidopropyl Dimethylamine | 2,20 | 2,20 | 2,20 |
| 13 | Parfum | 0,70 | 0,70 | 0,70 |
| 14 | Dimethicone + Cocamidopropyl Betaine + C12-15 Pareth-3 + Guar Hydroxypropyltrimonium Chloride | 4,60 | | |
| 15 | Dimethicone + C12-14 Sec-Pareth- 12 + Cocamidopropyl Betaine + C12-15 Pareth-3 | | 4,00 | 4,00 |
| | pH (90d) | 4,52 | 4,58 | 4,06 |
| | Viskosität (90d) | 3800/3350 | 4100/3300 | 4150/3450 |

### Handelsnamen der Rohstoffe:

- 1: Nipagin M, Clariant, 99.5%
- 2: Nipasol M, Clariant, 99.5%
- 5: Nacol 16-95, Sasol , 95%
- 6: 100366 Milchsäure 90, reinst DAB, Merck, 90%
- 8: Lanette 18, Cognis , 100%
Gamma -Oryzanol, Biesterfeld,
- 9: 98%
- 10: Jaguar Excel, Rhodia, 88%
- 11: Dehyton AB 30, Cognis, 31%
- 12: Tego Amid S18, Evonik Goldschmidt, 100%
DC 5-7139, 65% Dimethicone (enthält 0,5% Guar
- 14: Hydroxypropyltrimonium Chloride
- 15: DC 5-7175, 75% Dimethicone

Zubereitung 1 enthält die Siliconemulsion DC 5-7139 (Dimethicone). Der Gehalt an Guar Hydroxypropyltrimonium Chloride beträgt bevorzugt 0.4-0.6% (aktiv) im Silikon.

Guar Hydroxypropyltrimonium Chloride wurde zu den Formeln in Beispielen 2 + 3 zugesetzt.

Die Siliconemulsion DC 5-7175 enthält kein Guar Hydroxypropyltrimonium Chloride.

Die Zubereitungen 1 bis 3 wurden durch ein Expertenpanel bewertet (n=5), Tabelle 2.

**Tabelle 2**

| n=5 Experten | *(0.4%) Guar im Silikon* | | | | | | *0.022% Guar als Zusatz* | | | | | | *0.088% Guar als Zusatz* | | | | | |
|---|---|---|---|---|---|---|---|---|---|---|---|---|---|---|---|---|---|---|
| Formeln | 1 | 1 | 1 | 1 | 1 | Mittel wert 1 | 2 | 2 | 2 | 2 | 2 | Mit telwert 2 | 3 | 3 | 3 | 3 | 3 | Mittel wert 3 |
| Haarstruktur Strähne* | 2 | 4 | 2 | 2 | 2 | 2,4 | 2 | 4 | 1 | 2 | 2 | 2,2 | 3 | 4 | 2 | 2 | 2 | 2,6 |
| Verteilbarkeit | 7 | 8 | 8 | 8 | 7 | 7,6 | 7 | 8 | 9 | 6 | 7 | 7,4 | 7 | 7 | 8 | 6 | 7 | 7,0 |
| Gehalt Verteilen | 9 | 9 | 9 | 7 | 8 | 8,4 | 6 | 6 | 8 | 9 | 6 | 7,0 | 7 | 8 | 9 | 8 | 7 | 7,8 |
| Gleitvermögen Verteilen | 8 | 8 | 9 | 6 | 9 | 8,0 | 7 | 9 | 8 | 8 | 6 | 7,6 | 8 | 9 | 8 | 7 | 6 | 7,6 |
| Haarstruktur Verteilen | 8 | 9 | 7 | 9 | 9 | 8,4 | 7 | 8 | 6 | 8 | 7 | 7,2 | 8 | 9 | 7 | 8 | 7 | 7,8 |
| Quietschig beim Ausspülen | | | | | | | 1 | 1 | 1 | | 1 | 1 | | 1 | | | 1 | 1 |
| Quietschig, nasse Strähne | | | | | | | 1 | 1 | | | 1 | 1 | 1 | 1 | | | 1 | 1 |

### Protokoll fürs Expertenpanel:

*2Std. gebleichte 2g Haarsträhnen werden verwendet.
Haarsträhne mindestens 15min einweichen.
Hände mit Standardshampoo waschen.
Haarsträhne rausnehmen und mit dem groben Kammsegment und danach mit dem feinen Kammsegment kämmen.
Haarsträhne unter fließendem Wasser nass machen und ein Mal mit leichtem Druck ausstreichen.
Eine Spritze mit 0,4mL Produkt aufziehen. Den Inhalt der Spritze auf die Haarsträhne auftragen. Hände mit Wasser aus einem Pumpspender befeuchten.
Produkt 60s im angegebenen Takt (58bpm) das Produkt von oben nach unten verstreichen und nach 20s die Parameter: Verteilbarkeit, Gehalt, Gleitvermögen und Haarstruktur bewerten. Produkt 30s einwirken lassen und 50s ausspülen.

Zubereitung 1 war besser verteilbar, reichhaltiger, hatte ein höheres Gleitvermögen und führte zu einer weichern Haarstruktur im Vergleich zu Zubereitungen 2 und 3, in denen das kationische Polymer zusätzlich hinzugefügt wurde. Desweiteren gab es sensorische Nachteile beim Ausspülen der Haarsträhnen im Fall von Zubereitungen 2 & 3, weil die Haarsträhnen angefangen haben zu quietschen, was vom Verbraucher generell nach einer Spülungsanwendung als unvorteilhaft beschrieben wird.

### Weitere Rezepturen für Haarspülungen:

**Tabelle 3**

| wt% | **4** | **5** | **6** |
|---|---|---|---|
| METHYLPARABEN | 0,25 | 0,25 | 0,25 |
| PROPYLPARABEN | 0,10 | 0,10 | 0,10 |
| AQUA | Ad.100 | Ad.100 | Ad.100 |
| CETYL ALCOHOL | 2,50 | 2,50 | 2,50 |
| LACTIC ACID, 90% | 0,80 | 0,80 | 0,78 |
| BENZOPHENONE-4 | | 0,25 | 0,25 |
| SODIUM CHLORIDE | 0,01 | 0,01 | 0,01 |
| STEARYL ALCOHOL | 4,80 | 4,80 | 4,80 |
| ORYZANOL | 0,05 | 0,05 | 0,05 |
| COCO BETAINE | 0,85 | 0,85 | 0,85 |
| STEARAMIDOPROPYL DIMETHYLAMINE | 2,20 | 2,20 | 2,20 |
| DIMETHICONE + COCAMIDOPROPYLBETAINE + GUAR HYDROXYPROPYLTRIMONIUM CHLORIDE + C12-15 PARETH-3 | 4,60 | 4,60 | |
| DIMETHICONE + C12-14 SEC-PARETH-12 + COCAMIDOPROPYL BETAINE + C12-15 PARETH-3 | | | 4,40 |
| Parfum A | 0,70 | | |
| Parfum B | | 0,70 | 0,70 |
| pH (90d) | 4,17 | 4,12 | 4,20 |
| viscosity (90d) | 7150/4900 | 5150/3300 | 4800/2750 |

Zubereitungen 4 und 5 führten zu einer besseren Kämmbarketi und Pflegeleistung. Im Vergeleich zu Zubereitung 6.
Zubereitungen 4 und 5 enthielten 3.0% der Siliconemulsion mit Guar Hydroxypropyltrimonium Chloride, Zubereitung 6 enthielt 3.3% der Siliconemulsion ohne Guar Hydroxypropyltrimonium Chloride. Obwohl eine höhere Menge an Silikonöl in Zubereitung 6 vorhanden war, hat die Spülungsformel schlechtere Pflegeeigenschaften.

### Weitere Rezepturen für Haarspülungen:

**Tabelle 4**

| | | **7** | **8** | **9** | **10** | **11** | **12** | **13** |
|---|---|---|---|---|---|---|---|---|
| 1 | Methylparaben | 0,25 | 0,25 | 0,25 | 0,25 | 0,25 | 0,25 | 0,25 |
| 2 | Propylparaben | 0,10 | 0,10 | 0,10 | 0,10 | 0,10 | 0,10 | 0,10 |
| 3 | Aqua | Ad. 100 | Ad. 100 Ad. 100 | | Ad. 100 | Ad. 100 | Ad. 100 | Ad. 100 |
| 4 | Cetyl Alcohol | 2,50 | 2,00 | 2,00 | 2,00 | 2,00 | 2,00 | 2,00 |
| 5 | Citric Acid | | | | | | 0,005 0 | |
| 6 | Lactic Acid | 0,80 | 0,80 | 0,80 | 0,80 | 0,80 | | 0,80 |
| 7 | Sodium Chloride | 0,01 | 0,01 | 0,01 | 0,01 | 0,01 | | 0,01 |
| 8 | Stearyl Alcohol | 4,80 | 3,80 | 3,80 | 3,80 | 4,00 | 4,00 | 3,80 |
| 9 | Oryzanol | 0,05 | 0,05 | 0,05 | 0,05 | 0,05 | | 0,05 |
| 10 | Glycerin | | | | | | 5,00 | |
| 11 | Palmitamidopropyltrimonium Chloride | 0,42 | | | | | 3,32 | |
| 12 | Distearoylethyl Hydroxyethylmonium Methosulfate | | | | | | 2,86 | |
| 13 | Stearamidopropyl Dimethylamine | 2,00 | 2,00 | 2,00 | 2,00 | 2,20 | | 2,00 |
| 14 | Behentrimonium Methosulfate | | 0,50 | | | | | |
| 15 | Behentrimonium Chloride | | | | 1,00 | | | 1,00 |
| 16 | Cetrimonium Chloride | | | 2,00 | | | | |
| 17 | DIMETHICONE + CO-CAMIDOPROPYLBETAINE + GUAR HYDROXYPROPYLTRIMONIUM CHLORIDE + C12-15 PARETH-3 | 4,60 | 3,60 | 4,60 | 4,60 | 3,70 | 4,60 | 4,60 |
| 18 | Parfum | 0,70 | 0,70 | 0,70 | 0,70 | 0,70 | 0,70 | 0,70 |
| | pH (90d) | 4,20 | 4,10 | 4,25 | 4,55 | 4,55 | 4,20 | 4,55 |
| | viscosity (90d) | 7900/540 0 | 7100/4 900 | 7500/5 100 | 8100/ 5550 | 6500/4 100 | 5100/ 3500 | 8100/ 5550 |

### Handelsnamen der Rohstoffe:

- 1: Nipagin M, Clariant, 99.5%
- 2: Nipasol M, Clariant, 99.5%
- 4: Nacol 16-95, Sasol , 95%
- 8: Lanette 18, Cognis , 100%
Gamma-Oryzanol, Biesterfeld,
- 9: 98%
Varisoft PATC, Evonik Gold-
- 11: schmidt, 60%
Dehyquart F 75, Cognis,
- 12: 69.935%
Tego Amid S18, Evonik Gold-
- 13: schmidt, 100%
Incroquat Behenyl TMS-50,
- 14: Croda, 55%
Incroquat Behenyl TMC-25,
- 15: Croda, 25%
- 16: CTAC 6025 KC, KCI, 25%
- 17: DC 5-7139, 65% Dimethicone

## Patentansprüche

1. Kosmetische Haarbehandlungszubereitung vorgesehen für das Ausspülen nach der Anwendung mit kationischen Polymeren in Gehalten von 0,4 bis 0,7 Gew.-% vorhanden in einer hochmolekularen Silikonemulsion mit Partikelgrößen von 10 bis 50 Mikrometern.

2. Zubereitung nach Patentanspruch 1 **dadurch gekennzeichnet, dass** zusätzlich 0,1 bis 1 Gew.-% Oryzanol enthalten sind.

3. Zubereitung nach einem der vorangehenden Patentansprüche **dadurch gekennzeichnet, dass** als kationisches Polymer Guar Hydroxypropyltrimonium Chlorid verwendet wird.

4. Zubereitung nach einem der vorangehenden Patentansprüche **dadurch gekennzeichnet, dass** als kationisches Polymer Guar Hydroxypropyltrimonium Chloride mit einer mittleren Ladungsdichte von 0.2 bis 1 meq per gram vorhanden ist.

5. Zubereitung nach einem der vorangehenden Patentansprüche **dadurch gekennzeichnet, dass** die Silikonemulsion eine Viskosität 6000 -12000 mPas (Brookfield, Spindel 6, 50 RPM) hat.

6. Zubereitung nach einem der vorangehenden Patentansprüche **dadurch gekennzeichnet, dass** die Zubereitung frei von Cellulosederivaten ist.

7. Zubereitung nach einem der vorangehenden Patentansprüche **dadurch gekennzeichnet, dass** die Zubereitung nicht waschaktiv ist.

8. Zubereitung nach einem der vorangehenden Patentansprüche **dadurch gekennzeichnet, dass** die Zubereitung frei von anionischen Tensiden ist.

9. Zubereitung nach einem der vorangehenden Patentansprüche **dadurch gekennzeichnet, dass** die Zubereitung außer kationischen keine weiteren Polymere enthält.

10. Zubereitung nach einem der vorangehenden Patentansprüche **dadurch gekennzeichnet, dass** die Zubereitung amphotere Tenside enthält, bevorzugt Betaine, ganz besonders bevorzugt Coco-Betaine.
